# EUROPÄISCHE PATENTANMELDUNG

(11) **EP 3 409 265 A1**
(43) Veröffentlichungstag der Anmeldung: **05.12.2018**
(21) Anmeldenummer: 17173256.3
(22) Anmeldetag: 29.05.2017
(51) Int. Cl.: A61K 8/46, A61K 8/19, A61K 8/25, A61K 8/44, A61K 8/60, A61Q 19/00, C07C 309/17, C11D 1/28, C11D 11/04

(54) **VERFAHREN ZUR HERSTELLUNG VON HELLFARBIGEM DISALZ**

(71) Anmelder: BASF SE, 67056 Ludwigshafen am Rhein (DE)
(72) Erfinder: BRUNN, Claudia, 40597 Düsseldorf (DE); BEHLER, Ansgar, 46240 Bottrop (DE); ESKUCHEN, Rainer, 40764 Langenfeld (DE); RATHS, Hans-Christian, 40789 Monheim (DE)
(74) Vertreter: BASF IP Association

(57) **Zusammenfassung**

Vorgeschlagen wird ein Verfahren zur Herstellung hellfarbiger wässriger Tensid-Zusammensetzungen enthaltend
• 10 Gew.-% bis 60 Gew.-% - bezogen auf die gesamte Zusammensetzung - ein oder mehrerer **alpha-Sulfofettsäuredisalze (A)** der allgemeinen Formel (I),

R¹CH(SO₃M¹)COOM² (I),

worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine,
• **Wasser**,
wobei das Verfahren dadurch gekennzeichnet ist, dass man Fettsäuren der allgemeinen Formel (Ia),

R²CH₂COOH (Ia)

worin der Rest R² einen linearen oder verzweigten Alkyl- oder Alkenylrest mit 6 bis 18 C-Atomen bedeutet,
(i) einer Sulfonierung mit gasförmigem Schwefeltrioxid unterwirft,
(ii) das erhaltene saure Sulfonierprodukt - ggf. nach Alterung - neutralisiert und anschließend
(iii) einer Bleiche mit Wasserstoffperoxid unterwirft, wobei man die Bleiche bei einem pH-Wert oberhalb von 7 in einem Temperaturbereich von 80 bis 95 °C in Gegenwart von 50 bis 500 ppm - bezogen auf die gesamte Zusammensetzung - eines Stabilisators, ausgewählt aus der Gruppe Magnesiumoxid, Magnesiumsalze, Wasserglas, Nitrilotriessigsäure, Ethylendiamintetraessigsäure, Gluconsäure und ihre Salze durchführt.

## Beschreibung

### Gebiet der Erfindung

Die vorliegende Erfindung betrifft ein Verfahren zur Herstellung hellfarbiger wässrige Tensid-Zusammensetzungen mit einem Gehalt an alpha-Sulfofettsäuredisalzen.

### Stand der Technik

Anionische Tenside gehören zu den am weitesten verbreiteten grenzflächenaktiven Verbindungen und werden außer in Wasch- und Reinigungsmittel auch auf dem Gebiet der Kosmetik vielfältig eingesetzt. Übliche anionische Tenside, wie sie vor allem in der Kosmetik eingesetzt werden, sind die Salze von Alkylethersulfaten (Alkylpolyethersulfate, Fettalkoholpolyglycolethersulfate, verkürzt auch Ethersulfate). Sie zeichnen sich durch starkes Schaumvermögen, hohe Reinigungskraft, geringe Härte- und Fettempfindlichkeit aus und finden vielfach Verwendung zur Herstellung von kosmetischen Produkten wie beispielsweise Haarshampoos, Schaum- oder Duschbädern, aber auch in Handgeschirrspülmitteln.

Für viele aktuelle Anwendungen werden an anionische Tenside außer einer guten grenzflächenaktiven Wirkung weitere Anforderungen gestellt. Insbesondere in der Kosmetik ist eine hohe dermatologische Verträglichkeit erforderlich. Des Weiteren sind in der Regel ein gutes Schaumvermögen und eine angenehme Sensorik des Schaumes erwünscht. Des Weiteren besteht ein Bedarf an anionischen Tensiden, die zumindest teilweise aus biogenen Quellen und speziell auch nachwachsenden Rohstoffen hergestellt werden können. Tenside, die diese Anforderung ausgezeichnet erfüllen, sind alpha-Sulfofettsäure-Disalze, wie sie beispielsweise durch Sulfonierung von Fettsäuren mit gasförmigem Schwefeltrioxid nebst anschließender Neutralisation zugänglich sind.

Die Sulfonierung von gesättigten Fettsäuren zur Herstellung von alpha-Sulfofettsäuren ist seit langem aus der Literatur bekannt. Typischerweise erfolgt die Sulfonierung durch Umsetzung von Fettsäuren mit gasförmigem Schwefeltrioxid. Die dabei erhältlichen Produkte sich jedoch dunkelfarbig und in dieser Form nur begrenzt einsetzbar.

Verschiedene Patentschriften beschäftigen sich mit der Optimierung der Sulfonierbedingungen um möglichst von vorneherein die primäre Bildung von Farbträgern zu reduzieren, vergleiche zum Beispiel DE 4224735 und darin zitierte Dokumente. Die Verbesserung der Primärfarbe allein führt jedoch noch nicht zu einer akzeptablen Produktfarbe. Auf diese Weise können lediglich besser bleichbare Produkte bereitgestellt werden. Eine Nachbehandlung in Form einer Bleiche ist dennoch immer erforderlich.

Es ist bekannt, dass die Bleiche von Sulfonierprodukten von Fettsäure-Estern mittels Wasserstoffperoxid durchgeführt werden kann. Wobei die Bleiche ganz oder teilweise mit der noch nicht neutralisierten, sauren Rohsulfonsäure durchgeführt wird (vergleiche zum Beispiel DE 1179931, DE1234709, DE3319591). Untersuchungen der Anmelderin haben jedoch gezeigt, dass dies nicht ohne weiteres auf die Bleiche von Sulfonierprodukten von Fettsäuren übertragen werden kann, weil bei einer Bleiche der nicht neutralisierten, sauren Sulfonierprodukte von Fettsäuren eine Vergelung auftritt.

Untersuchungen der Anmelderin haben außerdem gezeigt, dass eine Bleiche der neutralisierten, wässrigen Paste von Sulfonierprodukten von Fettsäuren, die bei einem pH-Wert von 7 oder weniger mit Wasserstoffperoxid durchgeführt wird, keine akzeptable Produktfarben erreicht wird. Eine akzeptable Produktfarben würde den Einsatz sehr großer Mengen an Wasserstoffperoxid, eine lange Prozesszeit, sowie eine aufwändige Prozessüberwachung und -steuerung insbesondere zur Vermeidung von unkontrollierbarer Schaumbildung erfordern und außerdem zu einer unerwünschten Verdünnung, sprich Wassereintrag in das Produkt, führen.

### Beschreibung der Erfindung

Die Aufgabe der vorliegenden Erfindung hat darin bestanden, ein einfaches, sicheres, reproduzierbares und wirtschaftliches Verfahren zur Herstellung hellfarbiger wässriger Tensid-Zusammensetzungen enthaltend alpha-Sulfofettsäure-Disalze bereitzustellen, das auf der Sulfonierung von Fettsäuren mit gasförmigem Schwefeltrioxid nebst anschließender Neutralisation und Bleiche basiert, jedoch die oben genannten technischen Probleme einer Wasserstoffperoxidbleiche löst.

Unter dem Begriff "hellfarbig" wird im Rahmen der vorliegenden Erfindung verstanden, dass die wässrigen Tensidzusammensetzungen, nach Verdünnung auf einen Trockenrückstand von 4 Gew.-% - bezogen auf den Gesamtgehalt der verdünnten Lösung - eine Hazen-Farbzahl von 200 Hazen oder weniger, vorzugsweise 100 Hazen oder weniger, aufweisen. Die Verdünnung zur Farbmessung erfolgt dabei mit einer Mischung aus entionisiertem Wasser (95 Gew.-%) und Isopropanol (5 Gew.-%). Die Farbmessung erfolgt in einer 11mm Rundküvette in einem handelsüblichen Farbmessgerät (z.B. Lico 500 der Firma Hach Lange GmbH).

Gegenstand der Erfindung ist ein Verfahren zur Herstellung hellfarbiger wässriger Tensid-Zusammensetzungen enthaltend
- 10 Gew.-% bis 60 Gew.-% - bezogen auf die gesamte Zusammensetzung - ein oder mehrerer **alpha-Sulfofettsäuredisalze (A)** der allgemeinen Formel (I),

   R¹CH(SOₛM¹)COOM² (I),

   worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine,
- **Wasser,**
wobei das Verfahren dadurch gekennzeichnet ist, dass man Fettsäuren der allgemeinen Formel (Ia),

R²CH₂COOH (Ia)

worin der Rest R² einen linearen oder verzweigten Alkyl- oder Alkenylrest mit 6 bis 18 C-Atomen bedeutet,
(i) einer Sulfonierung mit gasförmigem Schwefeltrioxid unterwirft,
(ii) das erhaltene saure Sulfonierprodukt - ggf. nach Alterung - neutralisiert und anschließend
(iii) einer Bleiche mit Wasserstoffperoxid unterwirft, wobei man die Bleiche bei einem pH-Wert oberhalb von 7 in einem Temperaturbereich von 80 bis 95 °C in Gegenwart von 50 bis 500 ppm - bezogen auf die gesamte Zusammensetzung - eines Stabilisators, ausgewählt aus der Gruppe Magnesiumoxid, Magnesiumsalze, Wasserglas, Nitrilotriessigsäure, Ethylendiamintetraessigsäure, Gluconsäure und ihre Salze durchführt.

Wie oben ausgeführt enthalten die nach dem erfindungsgemäßen Verfahren erhaltenen Zusammensetzungen 10 Gew.-% bis 60 Gew.-% - bezogen auf die gesamte Zusammensetzung - ein oder mehrerer alpha-Sulfofettsäuredisalze (A) der allgemeinen Formel (I). In einer bevorzugten Ausführungsform enthalten die Zusammensetzungen 30 Gew.-% bis 40 Gew.-% - bezogen auf die gesamte Zusammensetzung - ein oder mehrerer alpha-Sulfofettsäuredisalze (A) der allgemeinen Formel (I).

In Formel (I) bedeuten die Reste M¹ und M² vorzugsweise Natrium (Na).

Wie oben ausgeführt beinhaltet das erfindungsgemäße Verfahren, dass man zunächst Fettsäuren mit gasförmigem Schwefeltrioxid umsetzt. Dabei setzt man das Schwefeltrioxid vorzugsweise in einer Menge ein, dass das molare Verhältnis von SO₃ zu Fettsäuren im Bereich von 1,0 : 1,0 bis 1,5 : 1,0 liegt. Die Fettsäuren werden dabei mit einer Vorlagetemperatur im Bereich von 70 bis 95 °C in den Reaktor eingebracht. Vorzugsweise wird das erhaltene flüssige Sulfonierprodukt nach der Sulfonierung in einer temperierten Nachreaktionsschlange für 5 bis 20 Minuten auf dieser Temperatur gehalten und gealtert.

Die so erhaltenen Rohprodukte, die saure Sulfonierprodukte darstellen, werden anschließend partiell oder vollständig neutralisiert, wobei eine vollständige Neutralisation mit wässriger NaOH bevorzugt ist.

Für die sich anschließende Bleiche mit Wasserstoffperoxid gelten die oben genannten Bedingungen, also:
1. Der pH-Wert liegt oberhalb von 7.
2. Die Temperatur liegt im Bereich von 80 bis 95 °C.
3. Die Bleiche erfolgt in Gegenwart von 50 bis 500 ppm - bezogen auf die gesamte Zusammensetzung - eines Stabilisators, ausgewählt aus der Gruppe Magnesiumoxid, Magnesiumsalze, Wasserglas, Nitrilotriessigsäure, Ethylendiamintetraessigsäure, Gluconsäure und ihre Salze.

Vorzugsweise wird die zu bleichende wässrige Tensid-Zusammensetzung in einem Reaktor vorgelegt, gerührt und temperiert und auf den gewünschten pH-Wert eingestellt. Der wässrigen Tensid-Zusammensetzung wird dann zunächst ein Stabilisator zugegeben, der unter den unten gewählten Bleichbedingungen einen schlagartigen Zerfall des Wasserstoffperoxids und somit unkontrollierbare Schaumbildung und Wärmefreisetzung verhindert. Vorzugsweise wird der Stabilisator in einer Menge von 200 bis 400 ppm - bezogen auf die gesamte Zusammensetzung - eingesetzt.

Die so vorbereitete Vorlage wird mit Wasserstoffperoxid (H₂O₂) versetzt. Das Wasserstoffperoxid wird dabei vorzugsweise als wässrige Lösung in einer handelsüblichen Konzentration von 30 bis 70 Gew.-%, vorzugsweise 30 bis 45 Gew.-% eingesetzt. Die Zudosierung des Wasserstoffperoxids erfolgt kontinuierlich in die vorgelegte Tensid-Zusammensetzung, vorzugsweise über ein unter den Flüssigkeitsspiegel abgetauchtes Rohr. Die Dosierrate wird insbesondere so gewählt, dass sich eine stationäre Peroxidkonzentration von <3000 ppm, vorzugsweise 100 bis 1000 ppm einstellt, wodurch ein ständiger Bleichfortschritt gewährleistet ist.

Die Bleiche erfolgt vorzugsweise bei einer Temperatur im Bereich von 85 bis 92 °C. Der pH-Wert der wässrigen Lösung wird während der Bleiche insbesondere auf einen Wert im Bereich von 10 - 11,5 und insbesondere 10,2 bis 10,7 eingestellt. Bei einem Abfall des pH-Wertes im Laufe des Bleichprozesses wird dieser nachgestellt. Die Einstellung des pH-Wertes erfolgt mit wässriger NaOH. Dabei beträgt die Konzentration der verwendeten NaOH-Lösung maximal 50 Gew.-%, vorzugsweise maximal 20 Gew.-%.

### Verwendung der Zusammensetzungen

Ein weiterer Erfindungsgegenstand ist die Verwendung der mit dem erfindungsgemäßen Verfahren erhältlichen Tensid-Zusammensetzungen für kosmetische Mittel, sowie Wasch- und Reinigungsmittel.

Im Hinblick auf kosmetische Mittel sind dabei insbesondere solche besonders bevorzugt, die in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten (etwa Zahnpasten, Mundwässern und dergleichen) vorliegen.

Im Hinblick auf Reinigungsmittel sind dabei insbesondere Mittel mit niedrigem pH-Wert zur Reinigung harter Oberflächen bevorzugt, wie Bad- und WC-Reiniger und dergleichen, sowie für Reinigungs- und/oder Duftgele zur Anwendung in sanitären Einrichtungen.

### Beispiele

### Eingesetzte Substanzen

### Eingesetztes Ausgangsmaterial:

ungebleichte, wässrige Zusammensetzung enthaltend ein alpha-Sulfofettsäure-Di-Natriumsalz technischer Qualität auf Basis von nativer C_{12/14}-Fettsäure (C_{12/14}-Gewichtsverhältnis 70:30); Zusammensetzung: Wassergehalt 46,5%, Trockenrückstand 53,5% bestehend aus alpha-Sulfofettsäure bzw. (Di-)Natriumsalz, C_{12/14}-Fettsäure bzw. Natriumsalz und Natriumsulfat ungefähr im Verhältnis 8:1:1, pH-Wert 5,1.

Die Farbe dieser Zusammensetzung war mit der unten genannten Methode nur nach nochmaliger Verdünnung um den Faktor 10 (entspricht 0,4 Gew.-% Trockenrückstand) messbar und lag dann bei 330 Hazen.

### Verwendete Messmethoden

**pH-Wert:** Unter Einsatz eines handelsüblichen pH-Meters, wurde der pH-Wert direkt in der Formulierung, also der wässrigen Tensidzusammensetzung, gemessen.

**Farbe:** Die wässrigen Tensidzusammensetzungen wurden mit einer Mischung aus entionisiertem Wasser (95 Gew.-%) und Isopropanol (5 Gew.-%) auf einen Trockenrückstand von 4 Gew.-% - bezogen auf den Gesamtgehalt der verdünnten Lösung - verdünnt. Der Farbwert dieser Lösung wurde in einer 11mm Rundküvette in einem handelsüblichen Farbmessgerät der Firma Hach Lange GmbH (Lico 500) bestimmt. Der Farbwert wird in der Einheit Hazen angegeben.

**Peroxidkonzentration:** Die Bestimmung der Peroxidkonzentration erfolgte mittels semi-quantitativen Teststreifen (MQuant von Fa. Merck) in wässriger Verdünnung angepasst an den Messbereich der Teststreifen.

### Ergebnisse der Bleichversuche

Die Angaben in Tabelle 1 und 2 sind jeweils bezogen auf eine Vorlagemenge von 100 kg.

**Tabelle 1:**

| Bleichbedingungen | **Beispiel 1** | **Vergleichsbeispiel 1** | **Vergleichsbeispiel 2** |
|---|---|---|---|
| Temperatur | 90°C | 90°C | 90°C |
| pH-Werte | 10,4-10,7 | 5,3-6,6 | 10,4 |
| Stabilisator | Magnesiumoxid 300ppm | - | - |
| Bleichmittel, insgesamt eingesetzte Menge | Wasserstoffperoxid (35%ig), 5,1kg | Wasserstoffperoxid (35%ig), 2,26 kg | Wasserstoffperoxid (35%ig), <0,5kg |
| Stationäre Peroxidkonzentration | 100-300 ppm | 1000-1500 ppm | - |
| pH-Stellmittel, insgesamt eingesetzte Menge | NaOH (20%ig), 16,8kg | NaOH (50%ig), 6,83kg | NaOH (20%ig), 12,0kg |
| Endfarbe und gesamte Bleichdauer | 88 Hazen nach 18 h, Abbruch da gewünschte Farbe erreicht | 399 Hazen nach 30 h, Abbruch wegen zu langer Dauer | - |
| Schaumneigung während des Bleichprozesses | gering | stark | extrem stark, Abbruch notwendig |

Im erfindungsgemäßen Beispiel 1 wurden die Prozessbedingungen so gewählt, dass ein guter, kontrollierter Bleichfortschritt gewährleistet war (siehe Abnahme der Produktfarbe über Prozessdauer, vergleiche Tabelle 2). Nach 18 Stunden wurde ein sehr hellfarbiges Produkt (88 Hazen) erhalten.

Im Vergleichsbeispiel 1 wurden Bedingungen gewählt, wie Sie dem Fachmann gemäß dem Stand der Technik naheliegend erscheinen. Es zeigte sich, dass in einem pH-Bereich unterhalb von 7, also im sauren pH-Bereich, selbst bei einer höheren stationären Peroxidkonzentration kein akzeptabler Bleichfortschritt erzielt werden kann (siehe Abnahme der Produktfarbe über Prozessdauer, vergleiche Tabelle 2). Nach 30 Stunden war das Produkt immer noch zu dunkel. Der Versuch wurde dann aufgrund zu langer Prozessdauer abgebrochen. Zudem erwies sich die auftretende Schaumbildung als problematisch.

Vergleichsbeispiel 2 zeigt, dass ohne Zugabe eines Stabilisators die Bleiche im alkalischen pH-Bereich gar nicht erst angefahren werden konnte. Zudosiertes Peroxid zerfiel sofort unter extrem starker Schaumbildung.

Die nachfolgende Tabelle 2 zeigt die gemessenen Produktfarbe in Abhängigkeit von der Zeit während des Bleichprozesses. Der in Beispiel 1 und Vergleichsbeispiel 1 jeweils erstgenannte Hazen-Wert ist derjenige, der im messbaren Bereich liegt.

**Tabelle 2**

| Bleichzeit | **Beispiel 1** Farbe (in Hazen) | **Vergleichsbeispiel 1** Farbe (in Hazen) |
|---|---|---|
| Nach 5 Stunden | 850 | |
| Nach 6 Stunden | 480 | |
| Nach 8 Stunden | 300 | |
| Nach 10 Stunden | 220 | |
| Nach 13 Stunden | 170 | |
| Nach 15 Stunden | 120 | |
| Nach 17 Stunden | 100 | 831 |
| Nach 18 Stunden | 88 | |
| Nach 20 Stunden | | 723 |
| Nach 23 Stunden | | 597 |
| Nach 28 Stunden | | 480 |
| Nach 30 Stunden | | 399 |

## Patentansprüche

1. Verfahren zur Herstellung hellfarbiger wässrige Tensid-Zusammensetzungen enthaltend
• 10 Gew.-% bis 60 Gew.-% - bezogen auf die gesamte Zusammensetzung - ein oder mehrerer **alpha-Sulfofettsäuredisalze (A)** der allgemeinen Formel (I),
R¹CH(SO₃M¹)COOM² (I)
worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamin,
• **Wasser,**
**dadurch gekennzeichnet, dass** man Fettsäuren der allgemeinen Formel (Ia),
R²CH₂COOH (Ia)
worin der Rest R² einen linearen oder verzweigten Alkyl- oder Alkenylrest mit 6 bis 18 C-Atomen bedeutet,
(i) einer Sulfonierung mit gasförmigem Schwefeltrioxid unterwirft,
(ii) das erhaltene saure Sulfonierprodukt - ggf. nach Alterung - neutralisiert und anschließend
(iii) einer Bleiche mit Wasserstoffperoxid unterwirft, wobei man die Bleiche bei einem pH-Wert oberhalb von 7 in einem Temperaturbereich von 80 bis 95 °C in Gegenwart von 50 bis 500 ppm - bezogen auf die gesamte Zusammensetzung - eines Stabilisators, ausgewählt aus der Gruppe Magnesiumoxid, Magnesiumsalze, Wasserglas, Nitrilotriessigsäure, Ethylendiamintetraessigsäure, Gluconsäure und ihre Salze durchführt.

2. Verfahren nach Anspruch 1, wobei der Rest R¹ in der Formel (I) einen gesättigten, linearen Alkylrest mit 10 bis 16 C-Atomen bedeutet, wobei in Bezug auf die Verbindungen (A) gilt, dass der Anteil der Verbindungen (A), bei denen der Rest R¹ ein Decyl- oder ein Dodecylrest ist, - bezogen auf die Gesamtmenge der Verbindungen (A) - bei 90 Gew.-% oder mehr liegt.

3. Verfahren nach Anspruch 1 oder 2, wobei die Reste M¹ und M² Natrium bedeuten.

4. Verfahren nach einem der Ansprüche 1 bis 3, wobei man im Bleichschritt (iii) als Stabilisator Magnesiumoxid einsetzt.

5. Verfahren nach einem der Ansprüche 1 bis 4, wobei man im Bleichschritt (iii) den Stabilisator in einer Menge von 200 bis 400 ppm einsetzt.

6. Verfahren nach einem der Ansprüche 1 bis 5, wobei man im Bleichschritt (iii) die Temperatur im Bleichschritt (iii) auf eine Wert im Bereich von 85 bis 92 °C einstellt.

7. Verfahren nach einem der Ansprüche 1 bis 6, wobei man im Bleichschritt (iii) den pH-Wert auf einen Wert im Bereich von 10 - 11,5 und insbesondere 10,2 bis 10,7 einstellt.

8. Verfahren nach einem der Ansprüche 1 bis 6, wobei man im Bleichschritt (iii) den pH-Wert auf einen Wert im Bereich von 10,2 bis 10,7 einstellt.

9. Verfahren nach einem der Ansprüche 1 bis 8, wobei man im Bleichschritt (iii) Wasserstoffperoxid in Form einer wässrigen Lösung in einer handelsüblichen Konzentration von 30 bis 70 Gew.-% und insbesondere 30 bis 40 Gew.-% einsetzt.

10. Verfahren nach einem der Ansprüche 1 bis 9, wobei man im Bleichschritt (iii) das Wasserstoffperoxid kontinuierlich unter Flüssigkeitsspiegel in die vorgelegte Tensid-Zusammensetzung zudosiert.

11. Verfahren nach Anspruch 10, wobei man im Bleichschritt (iii) die Zudosierungsrate des Wasserstoffperoxids so wählt, dass sich eine stationäre Peroxidkonzentration einstellt, die unterhalb von 3000 ppm liegt.

12. Verfahren nach Anspruch 10, wobei man im Bleichschritt (iii) die Zudosierungsrate des Wasserstoffperoxids so wählt, dass sich eine stationäre Peroxidkonzentration einstellt, die im Bereich von 100 bis 1000 ppm liegt

13. Verwendung der Zusammensetzungen, die erhältlich sind nach dem Verfahren gemäß einem der Ansprüche 1 bis 12, für kosmetische Mittel in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten.

14. Verwendung der Zusammensetzungen, die erhältlich sind nach dem Verfahren gemäß einem der Ansprüche 1 bis 12, für Mittel mit niedrigem pH-Wert zur Reinigung harter Oberflächen, wie Bad- und WC-Reiniger und dergleichen, sowie für Reinigungs- und/oder Duftgele zur Anwendung in sanitären Einrichtungen.
